# EUROPEAN PATENT SPECIFICATION

(11) **EP 1 115 386 B1**
(45) Date of publication and mention of the grant of the patent: **13.08.2003**
(21) Application number: 99949141.8
(22) Date of filing: 22.09.1999
(51) Int. Cl.: A61K 31/00, A61K 38/00, G01N 33/50, A61P 31/00, A61K 33/06, A61K 39/395

(54) **MEDICAMENT FOR THE TREATMENT OF INFECTION**
MEDIKAMENT ZUR BEHANDLUNG VON INFEKTIONEN
MEDICAMENT POUR LE TRAITEMENT D'INFECTIONS

(30) Priority: 22.09.1998 GB 9820658
(43) Date of publication of application: 18.07.2001
(73) Proprietor: MEDICAL RESEARCH COUNCIL, London W1N 4AL (GB)
(72) Inventor: WILSON, Robert, John, Macleod, Mill Hill, London NW7 2AB (GB); MULLINEAUX, Conrad, William, Barnet, Hertfordshire EN5 2LS (GB); LAW, Anna, Elizabeth, Margate, Kent CT9 5BY (GB)
(74) Representative: Campbell, Patrick John Henry
(86) International application number: GB9903180
(87) International publication number: WO00016758

(56) References cited:
- WO-A-98/35057
- MCCONKEY G.A. ET AL: "Inhibition of Plasmodium falciparum protein synthesis" THE JOURNAL OF BIOLOGICAL CHEMISTRY, vol. 272, no. 4, 1997, pages 2046-2049, XP002135059
- ROGERS M.J. ET AL: "The antibiotic micrococcin is a potent inhibitor of growth and protein synthesis in the malaria parasite" ANTIMICROBIAL AGENTS AND CHEMOTHERAPY, vol. 42, no. 3, March 1998 (1998-03), pages 715-716, XP000891786
- FICHERA M.E. ET AL: "A plastid organelle as a drug target in apicomplexan parasites" NATURE, vol. 390, 1997, pages 407-409, XP002135060
- DATABASE EMBL [Online] ACCESION NUMBER X95275, 29 June 1996 (1996-06-29) WILSON R.J.M. ET AL: "Complete gene map of the plastid-like DNA of the malaria parasite Plasmodium falciparum" XP002135064 -& J.MOL.BIOL. 261:155-172 (1996) XP000891792
- DATABASE EMBL [Online] ACCESION NUMBER D64004, 4 October 1995 (1995-10-04) KANEKO T. ET AL: "Sequence analysis of the genome of the unicellular Cyanobacterium Synechocystis sp. strain PCC6803. I. Sequence features in the 1 Mb region from map positions 64% to 92% of the genome" XP002135065 -& DNA RESEARCH 2, 153-166 (1995) XP000891791
- DATABASE EMBL [Online] ACCESION NUMBER AE000263, BLATTNER F.R. ET AL: "THE COMPLETE GENOME SEQUENCE OF ESCHERICHIA COLI K-12" XP002135066 -& SCIENCE 277, 1997, 1453-1474 XP002135063

## Description

### Field of the invention

This invention relates to inhibitors of the expression and activity of a gene product and screening methods for the identification of such inhibitors. It further relates to the use of said inhibitors for the manufacture of a medicament for the treatment of infection by an organism, such as a malaria parasite.

### Background to the invention

The malaria parasite and related Apicomplexans are unusual amongst non-photosynthetic organisms in that they possess two forms of organellar DNA, typically a property of plants. One form of organellar DNA is mitochondrial DNA and the other is a 35 kb circle of DNA present in a plastid. The plastid is thought to be of prokaryotic origin. The *ycf 24* gene^{1,2} which is present on the DNA of this plastid corresponds to a well conserved gene which is also found in red algal plastids³, various bacteria including *E. coli*^{*16*} and the cyanobacterium *Synechocystis* sp. strain PCC6803¹⁷. The plastid is a drug target^{18,19}.

The plastid genome of Apicomplexans⁴ is distinctive from, but broadly resembles that of the parasitic higher plant *Epifagus virginiana*⁵. It has been suggested that the *raison d'etre* for maintenance of vestigial plastid genomes is the small number of open reading frames they carry besides the highly selected set of genes dedicated to the organellar expression system⁶. Unlike Apicomplexans, *Epifagus* does not carry the *ycf 24* gene on its plastid genome, suggesting that either the vestigial organelles have different functions, or a stochastic process determines gene loss or transfer to the nucleus.

The high level of conservation of *ycf 24* (∼50% amino acid identity of the putative encoded peptide product with other orthologs)suggests that it is under strong selective pressure and likely to have a general function.

Organelle transfection is not available in Apicomplexans. Therefore "knockouts" can only be tested in surrogate organisms.

### Summary of the invention

The present inventors have disrupted *ycf 24* in both *Synechocystis* sp. strain pCC6803 and *E. coli*^{*7*}. Disruption of all the copies of the *ycf 24* gene in these organisms was found to be lethal. Disruption of some of the copies of *ycf 24* was found to give a 'ragged' phenotype in *Synechocystis* and appeared to cause a delay in or interfere in the process of septation during cell division. Thus *ycf 24* is an essential gene and appears to play a role in replication. The present invention is based on the inhibition of the expression or activity of the *ycf 24* product to inhibit the growth of an organism in vivo or ex vivo.

Thus the invention provides an antibody to the *ycf24* gene product or an antisense inhibitor capable of hybridising with *ycf24* mRNA for use in the treatment of infection of the human or animal body by an organism comprising said gene. Such an antibody or antisense inhibitor is useful in particular in the treatment of infection by a malaria parasite. The invention also provides a method of inhibiting the growth of an organism comprising the gene, which method comprises contacting the organism *ex vivo* with an antibody to the *ycf24* gene product or an antisense inhibitor capable of hybridising with *ycf24* mRNA.

Screens may be carried out to identify an inhibitor of *ycf24* gene product expression and/or activity. The invention provides a method of identifying a compound that inhibits the growth of an organism comprising the *ycf24* gene, the method comprising
(i) contacting a test compound with the *ycf 24* gene product, and
(ii) determining whether the test compound inhibits the activity of or binds to the product, any such binding or inhibition being indicative that the compound inhibits the growth of the organism.

The invention also provides a method of identifying a compound that inhibits the growth of an organism comprising the *ycf24* gene, the method comprising
(i) contacting a test compound with a test construct comprising a *ycf 24* promoter operably linked to a coding sequence,
(ii) determining whether the test compound inhibits expression driven by the promoter, any such inhibition being indicative that the compound inhibits the growth of the organism.

The invention further provides a pharmaceutical composition comprising an antibody to the *ycf24* gene product or an antisense inhibitor capable of hybridising with *ycf24* mRNA and a pharmaceutically acceptable carrier or diluent.

The invention also provides use of an antibody to the *ycf24* gene product or an antisense inhibitor capable of hybridising with *ycf24* mRNA for the manufacture of a medicament for treating or preventing infection by a unicellular organism comprising the *ycf24* gene in a patient.

### Brief description of the drawings

The invention is illustrated by the accompanying drawings in which:
Fig.1 shows phenotypic characters of wild type (WT), ragged transformant (R) and smooth mutant forms (S) of *Synechocystis* sp. : a) Colony shape and size; an enlargment is inserted in R, b) Scanning electron micrographs of whole cells - bar = 10µm, c) Cross-sections showing plaques - arrow, d) Whole cells with and without DAPI-staining. Arrows point to mis-segregation.
Fig.2 shows a Southern blot of DNA from *Synechocystis* sp., restricted with *Hind* III and hybridized with wild type (WT) *ycf* 24. The sizes expected for restriction fragments from WT (lane 4) and clones of heteroplasmic transformants (lanes 1-3) are given in the diagram below.
Fig.3 shows an effect of over-expression of *ycf 24* in *E.coli.* Fig. 3a shows nucleoids stained with DAPI in an uninduced culture of *E.coli* and Fig. 3b shows nucleoids stained with DAPI after induction of over-expression.

### Detailed description of the invention

The antibody or antisense of the invention can be used to inhibit the growth of a unicellular or multicellular organism in vivo or ex vivo. The organism may be a prokaryote or a eukaryote. The organism comprises the *ycf 24* gene. In the case of a eukaryote the nuclear genome of the organism may contain the *ycf 24* gene or the organism may contain an organelle which contains DNA comprising the *ycf 24* gene. Such an organelle is generally of prokaryotic origin, for example algal origin, and may be a plastid.

The organism may be a protozoa, such as an Apicomplexan, for example of clade plasmodium, piroplasm, sarcocystan or coccidium. The organism may be *Plasmodium falciparum*. The organism may be an alga. The organism may be a bacterium, such as a cyanobacterium, mycobacterium or *E. coli*. The organism may be free-living or a pathogen of humans and/or animals. It may be an intracellular or extracellular pathogen.

The *ycf 24* gene product is generally one which can be expressed from the coding region of:
(a) the polynucleotide sequence of SEQ ID NO: 1, 2 or 3 or a fragment thereof; or
(b) polynucleotides which can selectively hybridise to the coding region of (a) of a fragment thereof; or
(c) polynucleotides which, but for the degeneracy of the genetic code, would hybridise to (a) or (b).

The polynucleotides of (b) may selectively hybridise under conditions of medium to high stringency, for example 0.03M NaCl and 0.03M sodium citrate at from 50 to 60 degrees centigrade.

Thus the polynucleotide from which the *ycf 24* product is expressed generally has at least 70%, preferably at least 95, 97 or 99% sequence identity to the coding sequence of SEQ ID NO: 1, 2, or 3 over a region of at least 20, preferably at least 30, for instance at least 46, 60 or 100 or more contiguous nucleotides.

The *ycf 24* product generally has at least 50% sequence identity to the amino acid sequences shown is SEQ ID NO: 1, 2, or 3, preferably at least 80 or 90% and more preferably at least 95, 97 or 99% over a region of at least 20, preferably at least 30, for instance at least 46, 60, 100 or more contiguous amino acids. The term *ycf 24* product includes fragments of the amino acid sequences of SEQ ID NO: 1, 2 or 3 or of the homologous sequences discussed above. Suitable fragments will be at least 5, 10, 20 or 40 amino acids in size, for example at least 60 or 100 amino acids in size. Generally the *ycf 24* product has *ycf 24* product activity, and would generally be able to complement the activity of a naturally occurring *ycf 24* product.

An antibody to the *ycf 24* gene product or an antisense inhibitor capable of hybridising with *ycf 24* mRNA may be used in the present invention.

The invention provides a method of identifying a compound that inhibits the activity of *ycf 24* product. *ycf 24* product for use in this method can be obtained, for example, recombinantly by any method known in the art. The nucleotide sequences of the *ycf 24* genes of *Plasmodium falciparum, Synechocystis* Sp strain pCC6803 and *E.coli* are provided herein as SEQ ID NOs 1, 2 and 3 respectively. The *ycf 24* genes of other species may be identified by searching in databases for genes homologous to SEQ ID NO: 1, 2 or 3. Nucleotides with the same sequence as genes identified in a database can be made and expressed using routine methods which are known in the art. Alternatively *ycf* 24 genes can be identified by probing genomic or cDNA libraries with nucleotides comprising the *ycf 24* gene or a fragment of the *ycf 24* gene and then isolating and sequencing the nucleotides identified by the probe.

Any suitable format may be used for identifying the inhibitor of the activity. In the method the *ycf 24* product is generally in a suitable buffer, which includes any suitable biological buffer that can provide buffering capability at a pH conducive to the reaction requirements of the *ycf 24* product. The *ycf 24* product may be in conditions which are similar to the physiological conditions in which it occurs naturally.

In the method the *ycf 24* product may be in inside a cell or outside a cell. The cell may be the cell in which the *ycf 24* product naturally occurs, or a cell in which the *ycf 24* product is expressed recombinantly. The cell may be treated with agents which permeabilise the cell surface allowing test substances to enter the cell more readily. The *ycf 24* product used in the method may be in the form of an extract from such cells.

The method may determine whether a test compound is able to bind the *ycf 24* product, such as in a specific manner. This method may comprise allowing the *ycf 24* product to bind to a test compound and measuring the amount of binding that occurs, such as by measuring the amount of *ycf 24* bound to the compound. The amount of binding may be determined by measuring a characteristic of the *ycf* 24 product that changes upon binding, such as spectroscopic changes.

The assay format may be a 'band shift' system. This involves determining whether a test compound advances or retards the *ycf 24* product on gel electrophoresis relative to the *ycf 24* product in the absence of the compound.

The method may be a competitive binding assay. This determines whether the test compound is able to inhibit the binding of the *ycf 24* product to a substance which is known to bind to the *ycf 24* product, such as an antibody specific for product. This method allows test compounds to be identified which are 'analogues' of ycf 24 product.

The competitive binding system may comprise
(i) incubating the *ycf 24* product with a test compound and a labelled reference compound that is known to bind the product;
(ii) determining the amount of the labelled reference compound that is bound to the product; and
(iii) comparing the amount of bound labelled reference compound determined in step (ii) with the amount of said compound that binds to the product in the absence of the test compound;
wherein any reduction in the binding of the labelled reference compound in the presence of the test compound compared to the binding in the absence of the test compound shows that the test compound is competing with the reference compound for binding to the product. This indicates that the test compound could be an inhibitor of *ycf 24* product activity.

The amount of the labelled reference compound bound to the *ycf 24* product may be measured directly or indirectly. A direct measurement may be carried out by removing assay mixture containing the unbound labelled reference compound and measuring the amount of label that is in the product fraction. Alternatively, the amount of labelled reference compound bound to the product could be determined indirectly by measuring the amount of label remaining in the assay solution after removal of the product fraction, which will be inversely related to the amount that has bound to the product.

In a competitive binding assay system, the *ycf 24* product may be immobilised on a solid support or may be in solution. The use of immobilised product has the advantage that, after the binding reaction is complete, the product/labelled reference compound complex may be separated from the labelled reference compound that remains in solution by simply removing the solution away from the solid support. If, on the other hand, the product is not immobilised during the assay but rather is in solution, then it will generally be necessary to devise a means for separating the product/labelled reference compound complex from the uncomplexed reference compound before measuring the amount of label. Such separation could be achieved, for example, by precipitating the product using an antibody to the product or by using a non-specific precipitation technique.

Suitable labels for use in the assay systems according the invention are well-known in the art. The reference compound which is know to bind the *ycf 24* product may be an antibody.

An antibody to *ycf 24* product may be produced by raising antibody in a host animal against the whole *ycf 24* product or an antigenic epitope thereof (hereinafter "the immunogen"). Methods of producing monoclonal and polyclonal antibodies are well-known. A method for producing a polyclonal antibody comprises immunising a suitable host animal, for example an experimental animal, with the immunogen and isolating immunoglobulins from the serum. The animal may therefore be inoculated with the immunogen, blood subsequently removed from the animal and the IgG fraction purified. A method for producing a monoclonal antibody comprises immortalising cells which produce the desired antibody. Hybridoma cells may be produced by fusing spleen cells from an inoculated experimental animal with tumour cells (Kohler and Milstein, Nature 256, 495-497, 1975).

An immortalized cell producing the desired antibody may be selected by a conventional procedure. The hybridomas may be grown in culture or injected intraperitoneally for formation of ascites fluid or into the blood stream of an allogenic host or immunocompromised host. Human antibody may be prepared by *in vitro* immunisation of human lymphocytes, followed by transformation of the lymphocytes with Epstein-Barr virus.

For the production of both monoclonal and polyclonal antibodies, the experimental animal is suitably a goat, rabbit, rat or mouse. If desired, the immunogen may be administered as a conjugate in which the immunogen is coupled, for example via a side chain of one of the amino acid residues, to a suitable carrier. The carrier molecule is typically a physiologically acceptable carrier. The antibody obtained may be isolated and, if desired, purified.

Any suitable assay format may be used for identifying an inhibitor of *ycf 24* expression. As mentioned above, the invention provides a method of identifying a compound that inhibits the growth of an organism comprising the *ycf 24* gene, the method comprising
(i) contacting a test compound with a test construct comprising a *ycf 24* promoter operably linked to a coding sequence, and
(ii) determining whether the test compound inhibits expression driven by the promoter, any such inhibition being indicative that the compound inhibits the growth of the organism.

Generally this method is carried out in conditions which in the absence of the test compound lead to expression of the coding sequence from the test construct. The test construct may also comprise other untranscribed or untranslated regions of the *ycf 24* gene. The coding sequence typically encodes a protein that is able to act as a reporter of expression. The assay may be carried out in a cell which harbours the nucleic acid. The substance may be tested with any other known promoter to test the possibility that the test substance is a general inhibitor of gene expression.

Any reporter polypeptide may be used, for example GUS or GFP. GUS is assayed by measuring the hydrolysis of a suitable substrate, for example 5-bromo-4-chloro-3-indolyl-β-D-glucoronic acid (X-gluc)or 4-methylumbelliferyl-β-glucuronide (MUG). The hydrolysis of MUG yields a product which can be measured fluorometrically. GFP is quantified by measuring fluorescence at 590nm after excitation at 494nm. These methods are well known to those skilled in the art.

Alternatively the coding sequence may be the *ycf 24* coding sequence itself, or a fragment of this sequence. The expression of the *ycf 24* product may be measured by for example, Northern/RNA blotting, Western/antibody blotting, RNA in situ hybridization or immunolocalisation. Thus the method for identifying an inhibitor of expression of *ycf 24* product may be performed on a cell which naturally harbours the *ycf 24* gene, or an extract from such a cell.

Suitable candidate substances which may be inhibitors and which can be tested in the methods discussed above include antibody products (for example, monoclonal and polyclonal antibodies, single chain antibodies, chimeric antibodies and CDR-grafted antibodies) which are specific for *ycf 24* product. Furthermore, combinatorial libraries, defined chemical identities, peptide and peptide mimetics, oligonucleotides and natural product libraries, such as display libraries (e.g. phage display libraries) may also be tested. The candidate substances may be chemical compounds. Batches of the candidate substances may be used in an initial screen of, for example, ten substances per reaction, and the substances of batches which show inhibition tested individually.

### Antisense inhibitors

As noted above the expression of *ycf 24* in a cell may be reduced by the presence in that cell of a compound which can bind to the *ycf 24* mRNA. Therefore a polynucleotide which is capable of hybridizing to *ycf 24* mRNA can constitute an appropriate inhibitor of *ycf 24* expression.

The polynucleotide may be antisense to the *ycf 24* mRNA. Such a polynucleotide may be capable of hybridising to *ycf 24* mRNA and may thus inhibit the expression of *ycf 24* product by interfering with one or more aspects of *ycf 24* mRNA metabolism including processing, translation and metabolic turnover.

The antisense polynucleotide may be RNA. This may hybridise to the *ycf 24* mRNA to form an RNA-RNA duplex which may cause direct inhibition of translation and/or the destabilization of the target message, for example, rendering susceptibility to nucleases.

Such a polynucleotide may hybridize to all or part of the *ycf 24* mRNA. Typically the antisense polynucleotide hybridizes to the ribosome binding region or the coding region of the *ycf 24* mRNA. The polynucleotide may be complementary to all of or a region of the *ycf 24* mRNA. For example, the polynucleotide may be the exact complement of all or a part of *ycf 24* mRNA. However, absolute complementarity is not required and polynucleotides which have sufficient complementarity to form a duplex having a melting temperature of greater than 20°C, 30°C or 40°C under physiological conditions are particularly suitable for use in the present invention. The polynucleotide may be a polynucleotide which hybridises to the *ycf 24* mRNA under conditions of medium to high stringency such as 0.03M sodium chloride and 0.03M sodium citrate at from about 50 to about 60 degrees centigrade.

It is preferred that the polynucleotide hybridizes to all or part of the region of the *ycf 24* mRNA corresponding to the coding sequence defined by nucleotides 26 to 1435 of SEQ ID NO:1. However, the polynucleotide may hybridise to all or part of the 5'- or 3'-untranslated region of the mRNA. The polynucleotide will typically be from 6 to 40 nucleotides in length. Preferably it will be from 12 to 20 nucleotides in length. The polynucleotides may be at least 40, for example at least 60 or at least 80, nucleotides in length and up to 100, 200, 300, 400, 500, 600 or 700 nucleotides in length or even up to a few nucleotides, such as five or ten nucleotides, shorter than SEQ ID NO: 1, 2 or 3.

When the polynucleotide is an antisense RNA it may be expressed in a cell from a recombinant replicable vector. Such a replicable vector comprises a polynucleotide which when transcribed gives rise to antisense RNA.

Thus the antisense polynucleotide may be provided by delivering such a vector to the cell and allowing transcription from the vector to occur. Such a vector is also understood to an inhibitor of *ycf 24* expression in this specification. Generally on the vector the polynucleotide giving rise to the antisense RNA is operably linked to a control sequence which is capable of providing for the transcription of the polynucleotide giving rise to the antisense RNA. The term "operably linked" refers to a juxtaposition wherein the components described are in a relationship permitting them to function in their intended manner. A control sequence "operably linked" to a sequence giving rise to an antisense RNA is ligated in such a way that transcription of the sequence is achieved under conditions compatible with the control sequences.

The vector may be for example, a plasmid or virus vector provided with an origin of replication, optionally a promoter for transcription to occur and optionally a regulator of the promoter. The vector may contain one or more selectable marker genes, for example an ampicillin resistance gene in the case of bacterial plasmid or a neomycin resistance gene for a mammalian vector. Vectors may be used in vitro, for example for the production of antisense RNA or used to transfect or transform a host cell. The term 'host cell' refers either to the organism whose growth it is desired to inhibit, or a human or animal cell which can be infected by the organism. Thus the host cell may be an infected cell. The vector may also be adapted to be used in vivo, for example in a method of gene therapy.

Promoters/enhancers and other expression regulation signals may be selected to be compatible with the host cell for which the expression vector is designed. For example, mammalian promoters, such as b-actin promoters, may be used. Tissue-specific promoters, in particular neuronal cell specific promoters (for example the tyrosine hydroxylase (TH), L7, or neuron specific enolase (NSE) promoters), are especially preferred. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, herpes simplex virus promoters or adenovirus promoters. All these promoters are readily available in the art. Preferred promoters are tissue specific promoters such as the casein gene promoter.

The vector may further include sequences flanking the polynucleotide giving rise to antisense RNA which comprise sequences homologous to eukaryotic genomic sequences, preferably mammalian genomic sequences, or viral genomic sequences. This will allow the introduction of the polynucleotides of the invention into the genome of eukaryotic cells or viruses by homologous recombination. In particular, a plasmid vector comprising the expression cassette flanked by viral sequences, for example HSV1 or HSV2 sequences, can be used to prepare a viral vector, for example an HSV vector, suitable for delivering the polynucleotides of the invention to a mammalian cell. Other examples of suitable viral vectors include retroviruses, including lentiviruses, adenoviruses and adeno-associated viruses. Gene transfer techniques using these viruses are known to those skilled in the art. Retrovirus vectors for example may be used to stably integrate the polynucleotide giving rise to the antisense RNA into the host genome. Replication-defective adenovirus vectors by contrast remain episomal and therefore allow transient expression.

Antisense polynucleotides may be chemically modified. This may enhance their resistance to nucleases and may enhance their ability to enter cells. For example, phosphorothioate oligonucleotides may be used. Other deoxynucleotide analogs include methylphosphonates, phosphoramidates, phosphorodithioates, N3'P5'-phosphoramidates and oligoribonucleotide phosphorothioates and their 2'-O-alkyl analogs and 2'-O-methylribonucleotide methylphosphonates.

Alternatively mixed backbone oligonucleotides (MBOs) may be used. MBOs contain segments of phosphothioate oligodeoxynucleotides and appropriately placed segments of modified oligodeoxy- or oligoribonucleotides. MBOs have segments of phosphorothioate linkages and other segments of other modified oligonucleotides, such as methylphosphonate, which is non-ionic, and very resistant to nucleases or 2'-O-alkyloligoribonucleotides.

An inhibitor of *ycf 24* expression and/or activity is one which produces a measurable reduction in *ycf 24* expression and/or activity in the methods described above. Preferred substances are those which inhibit *ycf 24* expression and/or activity by at least 10%, at least 20%, at least 30%, at least 40% at least 50%, at least 60%, at least 70%, at least 80%, at least 90%, at least 95% or at least 99% at a concentration of the inhibitor of 1µg ml⁻¹, 10µg ml⁻¹, 100µg ml⁻¹, 500µg ml⁻¹, 1mg ml^{-1,} 10mg ml⁻¹ or 100mg ml⁻¹. The percentage inhibition represents the percentage decrease in expression/activity in a comparison of assays in the presence and absence of the test substance. Any combination of the above mentioned degrees of percentage inhibition and concentration of inhibitor may be used to define an inhibitor of the invention, with greater inhibition at lower concentrations being preferred.

The inhibitor inhibits the growth of the organism. Generally the inhibitor will decrease the rate of cell division of the organism. Generally the inhibitor will decrease the number of divisions that occur in a unit of time by at least 10%, 20%, 30%, 40%, 50%, 60%, 70%, 80%, 90% or 100% at a concentration of inhibitor of 1µg ml⁻¹, 10µg ml⁻¹, 100µg ml⁻¹, 500µg ml⁻¹, 1mg ml⁻¹' 10mg ml⁻¹ or 100mg ml⁻¹.

If the organism is one in which the *ycf 24* gene is present in an organelle then the inhibitor generally decreases the rate of division of that organelle. The inhibitor may also disrupt cell or organelle division in other ways, such as causing abnormal septation or mis-segregation of DNA. The inhibitor may also disrupt the structure of the organism, such as giving the organism a 'ragged' appearance. The inhibitor may cause the death of the organism.

Candidate substances which show activity in assays such as those described herein can then be tested on the organism in question. The inhibitor may or may not be toxic towards humans or animals. The inhibitor may thus be used as an antibiotic.

The antibodies or antisense inhibitors described above may be used for the manufacture of a medicament to treat infections by an organism and, in particular, malaria. The condition of a patient suffering from an infection can therefore be improved by administration of such an antibody or inhibitor. A therapeutically effective amount of such an antibody or inhibitor may be given to a human patient in need thereof.

The formulation of antibody or antisense inhibitor for use in preventing or treating infection by an organism will depend upon factors such as whether a pharmaceutical or veterinary use is intended. In order to be administered to a patient, the antibody or antisense inhibitor will be provided in the form of a pharmaceutical composition containing the antibody or inhibitor and a pharmaceutically acceptable carrier or diluent. Suitable carriers and diluents include isotonic saline solutions, for example phosphate-buffered saline. Typical oral dosage compositions include tablets, capsules, liquid solutions and liquid suspensions. For example it may be formulated for parenteral, intravenous, intramuscular, subcutaneous, transdermal or oral administration.

The dose of antibody or antisense inhibitor may be determined according to various parameters, especially according to the antibody or inhibitor used; the age, weight and condition of the patient to be treated; the route of administration; and the required regimen. A physician will be able to determine the required route of administration and dosage for any particular patient.

The antibody or antisense inhibitor may be used in either the treatment of an existing infection by the organism or in the prevention of such an infection from occurring in the first place.

Vectors giving rise to antisense RNA of the invention may be administered directly as a naked nucleic acid construct. Uptake of naked nucleic acid constructs by mammalian cells is enhanced by several known transfection techniques, for example those including the use of transfection agents. Example of these agents include cationic agents (for example calcium phosphate and DEAE-dextran) and lipofectants (for example lipofectam™ and transfectam™ ). These agents may also enhance the uptake of the constructs by the organism.

The pharmaceutical composition may be formulated to aid delivery of the antibody or antisense inhibitor to the organism whose growth is to be inhibited. In particular it may be formulated to deliver the antibody or inhibitor to the cell type which is infected or to the compartment in the human or animal cell which is infected by the organism. It may be formulated to deliver the antibody or inhibitor to the organelle in the organism where *ycf 24* product or mRNA is present.

When the pharmaceutical composition comprises viral vector for gene therapy it may be administered in such a way that the vector is incorporated into cells at an appropriate area. The amount of virus administered is in the range of from 10⁴ to 10⁸ pfu, preferably from 10⁵ to 10⁷ pfu, more preferably about 10⁶ pfu for herpes viral vectors and from 10⁶ to 10¹⁰ pfu, preferably from 10⁷ to 10⁹ pfu, more preferably about 10⁸ pfu for adenoviral vectors. When injected, typically 1-2 ml of virus in a pharmaceutically acceptable suitable carrier or diluent is administered. When the polynucleotide is administered as a naked nucleic acid, the amount of nucleic acid administered is typically in the range of from 1 µg to 10 mg.

Where the polynucleotide giving rise to the antisense RNA is under the control of an inducible promoter, it may only be necessary to induce gene expression for the duration of the treatment. Once the condition has been treated, the inducer is removed and expression of the polypeptide of the invention ceases. This will clearly have clinical advantages. Such a system may, for example, involve administering the antibiotic tetracycline, to activate gene expression via its effect on the tet repressor/VP16 fusion protein.

The use of tissue-specific promoters will be of assistance in the treatment of disease using the vectors of the invention. It will be advantageous to be able express inhibitors of *ycf 24* in only the relevant affected cell types, especially where such inhibitors are toxic when expressed in other cell types.

When the inhibitor is used to inhibit the growth of an organism ex vivo the organism may or may not be a pathogen of humans or animals. The inhibitor may be used to treat land, water or food containing the organism.

The following Examples illustrate the invention:

### Example 1

### Gene disruption of ycf 24, transformation and selection

A central segment of the wild type gene (slr0074) from *Synechocystis* sp., strain pCC6803, (delineated by two *Hin*dIII sites ∼1.0 kb apart) was amplified from genomic DNA by PCR using two oligonucleotide primers based on the known sequence (Accession no. S76598): 5' CTC CGC CCC TAA GCA AAG TAA GAA A and 5' TGA TCC TCG CCA ATT TTG GAA GTG GA. The amplified fragment was restricted with *Hin*dIII and cloned into pUC9 to give pUC9/slr . The sequence of both ends of the insert was confirmed in recombinants grown in Epicurian SURE cells. To disrupt the gene, a kanamycin resistance gene (*k*^{r}) isolated from a modified bluescript vector (pBSHdSp1, was blunt-ended with the Klenow fragment and cloned into a unique *Xba*I site in the *ycf* 24 insert to give pUC9/slr/*k*^{r}. Recombinants in XL1-blue MRF' cells were selected using kanamycin. Disruption of the *ycf* 24 insert was confirmed by sequencing the junction regions.

Wild type (WT) cells of *Synechocystis* sp. strain PCC 6803 were transfected by incubation with light at 30°C for 4 hr with vector DNA carrying the disrupted *ycf* 24 gene (1-5 µg in 10 µl water). Selection and growth were carried out on BG11 plates under photo-autotrophic conditions at 30°C. Several rounds of replication were allowed over 3 days before recombinants were selected by overlaying with kanamycin (50 µg/ml). This killed most of the cells but transformants emerged after a delay. Every 10-14 days thereafter, transformants were picked and re-plated to allow segregation of mutant cells. For growth comparisons in the absence of selection, WT cells were plated at the same time on BG11 (their growth was inhibited totally by kanamycin at 50 µg/ml).

### Example 2

### Gene Disruption in E.coli

Disruption of *ycf* 24 in *E. coli* was carried out along similar lines, but in this case disruption was with the *aad*A gene for streptomycin resistance (*S*^{r}). *ycf* 24 from *E.* *coli* was amplified using primers based on the accession no. D90811 with added terminal restriction sites: 5' GAG CTC GGA ATT CGC ATG TGG CTG TGG CGA AAG and 3' GAG CTC GGG ATC CTT ATC CGA CGC TGT GTT CAA G. The *E.coli aad*A gene was introduced at *a Bsg*1 restriction site near the centre of *ycf* 24 and cloned into the bluescript vector pBSKS⁺ The construct was linearized for transformation and to allow homologous recombination in *E. coli* LE392 host cells. The linearized recombinant plasmid pBSKS⁺/*ycf*24/*S*^{r} was transfected into *E. coli* by heat shock but no homologous recombinants were found from the primary normal size colonies; a few antibiotic resistant colonies were recovered that carried circular episomes likely to have arisen from re-ligation of the construct. After 2 days incubation at 37°C small colonies were observed. Cells from these were found to be elongated (at least x3 length of host cells) but they survived for only a few generations. PCR from these colonies to determine if a "knock-out" had occurred was unsuccessful.

### Example 3

### Characterising the Synechocytis transformants

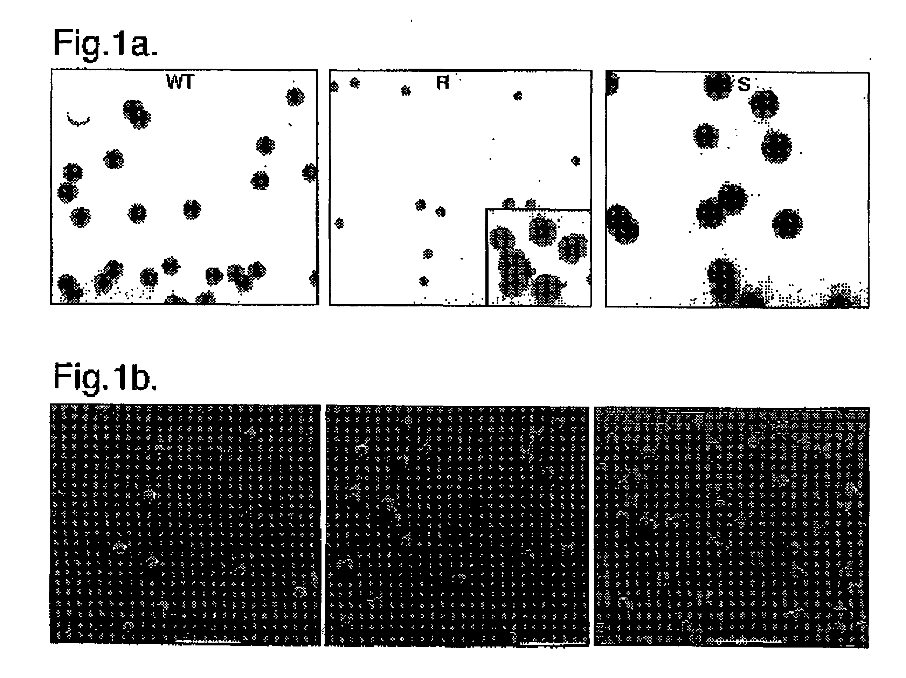

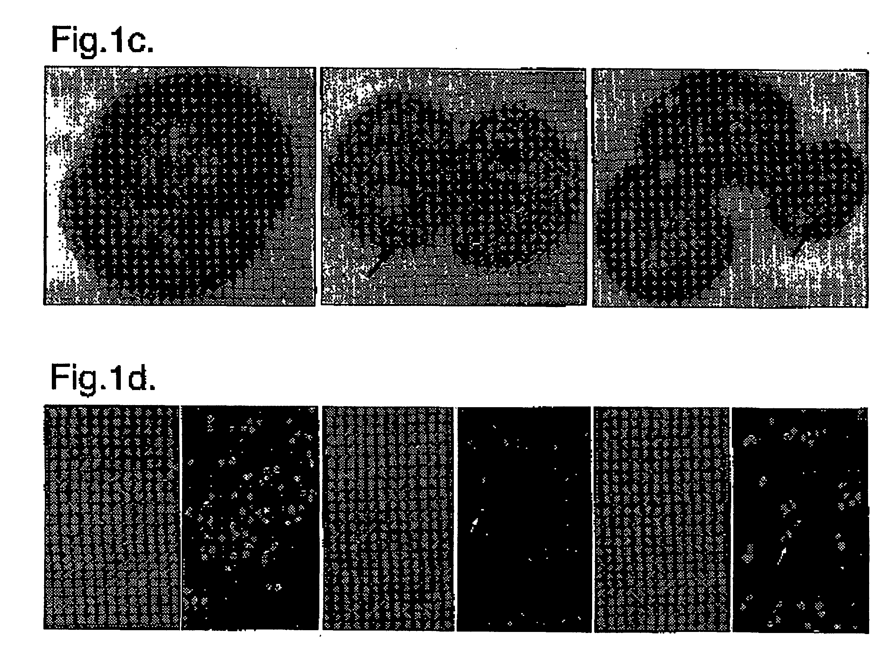

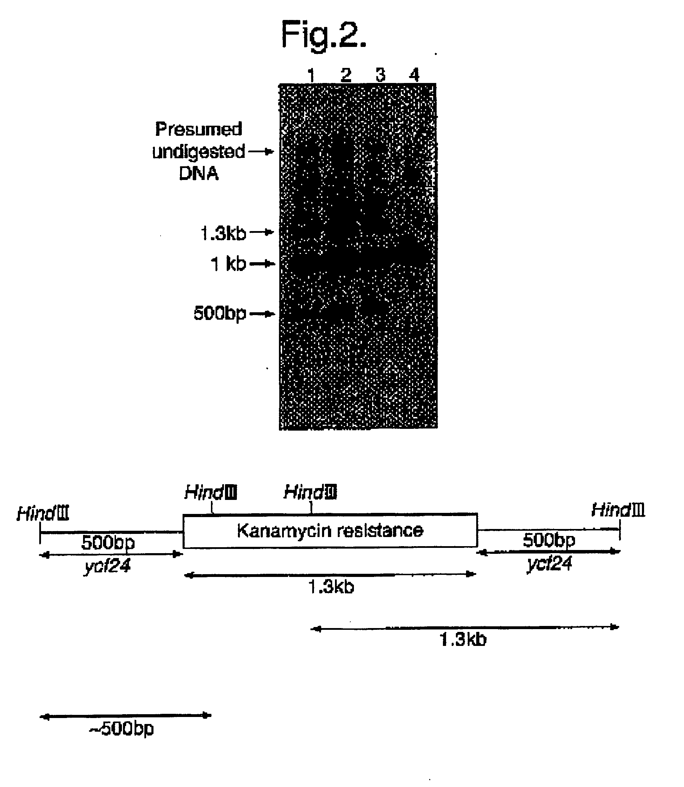

WT *Synechocystis* and transfectants carrying an homologous form of *ycf* 24 interrupted by a kanamycin resistance gene (*k*^{r}) were plated on BG-11 agar. The WT cells formed colonies visible to the naked eye within 4 days, whereas it took 2 weeks for transformants to appear under selection with kanamycin (50 µg ml⁻¹). Colonies of transformants were small and "ragged" in outline in contrast to the larger, smooth-contoured WT colonies (Fig. 1a). DNA extracted from cloned transformants was hybridized in Southern blots with WT *ycf* 24 and found to be heteroplasmic, the copy numbers of the disrupted and WT forms of *ycf* 24 being approximately equal as judged from the intensity of the hybridization signals (Fig. 2). Relaxation of kanamycin selection on heteroplasmic transformants resulted in vigorously growing, smooth-contoured colonies - "revertants", confirming that the heteroplasmic state was growth-limiting. These observations were confirmed by additional transformations, including one using disruption with a spectinomycin resistance gene instead of kanamycin. Transformants homoplasmic for the disrupted form of *ycf* 24 were never recovered, even after prolonged selection with kanamycin at 50 or 100 µg ml⁻¹ and homoplasmy is inferred to be lethal.

Longer-term selection of "ragged" transformants on kanamycin also gave rise to rapidly growing colonies with smooth contours. However, hybridization on Southern blots showed these remained heteroplasmic for the disrupted gene and we refer to these as "smooth" suppressors. DNA from cloned "smooth" suppressors was used to transform both WT and "ragged" heteroplasmic cells. In both cases, kanamycin selection yielded mostly "ragged" colonies but small numbers (<1%) of smooth, rapidly growing colonies were found, unlike in the original transfections.

The cells were examined by electron microscopy. Pelleted cells were pre-fixed in 0.5% neutral glutaraldehyde in 100mM cacodylate buffer, pH 7.0 for 30 min at room temperature. After gentle centrifugation, the pelleted cells were fixed in 2-3% glutaraldehye buffer and thin sections were prepared using standard procedures. For scanning electron microscopy (SEM), cells were spread on glass slips coated in poly-L-lysine (100 µg ml⁻¹) and fixed in 2% glutaraldehyde. Post fixation samples were washed in ethanol and rehydrated by immersion in hexamethyl-disilizane. After overnight dessication, samples were sputter-coated with 20 nm gold (for sample conductivity) and examined by SEM.

Electron micrographic sections showed ∼80% of cells from "ragged" colonies (>100 cells examined) had electron-translucent plaques surrounding the phycobilisomes in the thylacoid membranes (Fig. 1c). The number of cells containing plaques fell to ∼45% upon relaxation of kanamycin selection pressure, coincident with reversion to normal colony morphology. Because similar plaques were found in ∼4% WT cells, we suggest they indicate "sick" cells rather than a direct phenotypic change following disruption of ycf 24.

By contrast, examination of the gross morphology of cells from "ragged" colonies by scanning electron microscopy showed a significant accumulation of cells (∼35%) in the last stages of cell division (p = <0.001), suggesting a defect in cytokinesis (Table 1 and Fig.1b). Relaxation of kanamycin selection again saw the proportion of dividing cells in the transformed cell population return to one resembling WT . A high proportion (∼60%) of the "smooth" suppressor cells also accumulated at a late stage of cytokinesis, with occasional small chains of incompletely separated cells. Smooth suppressors still contained electron-opaque plaques and electron micrographic sections confirmed that suppression increased the frequency of abberant septation in dividing cells.

**Table 1**

| % Fission | | | | |
|---|---|---|---|---|
| Cell type ^{*1} | Early stage | Late stage | Total | P (X²) ^{*3} |
| WT | 10 | 9^{±}1 | 19 | - |
| Ragged | 21 ^{±}6 | 34^{±}1 | 55 | <0.001 |
| Revertant ^{*2} | 8 | 4 | 12 | 0.9 |
| Smooth | 20 | 41 | 61 | <0.001 |

| | | | | |
|---|---|---|---|---|
| ^{*1} Nos. cells counted = >100/group (mean data from 2 exp.) | | | | |
| ^{*2} Ragged transformants grown without kanamycin selection | | | | |
| ^{*3} Chi-square comparison with WT | | | | |

DNA was stained with 4',6-diamindino-2-phenylindole (DAPI) at 0.01µg ml⁻¹, following a protocol similar to that described¹⁵. When stained with DAPI, the nucleoids of WT cells were highly uniform in appearance (Fig.ld). Most WT cells (∼82%) were not in division and only a few dead cells (unstained) were present. DAPI-staining of cells from "ragged colonies' was much more variable, ranging from cells that looked like WT to a large number (∼50%) of dead or dying cells that stained less intensely. The latter included >50% of those in division. Although nucleoid segregation had occurred in many of these cells, in ∼40% the DNA had mis-segregated (ie. one daughter cell was without DNA or carried less DNA as judged by staining intensity); altered autofluorescence under UV light indicated these were dying cells. DAPI-stained "smooth" suppressants also were distinctive. About half of the cells (both dividing and non-dividing) carried normal amounts of DNA but occasional cells either had no DNA (∼1% dead), had undergone asymmetric DNA segregation (∼1%), or had about twice as much DNA (∼1%) as WT. The cells in this last group were larger than the rest (Fig. 1d). Coulter counter analysis of the size distribution of the different populations (data not shown) confirmed that the numbers of small and large cells had substantially increased in both the "ragged" and smooth isolates compared with WT. The amount of DNA per cell corresponded generally with cell size, excepting dead cells.

### Example 4

### Over-expression of vcf 24 in E.coli

The effect of over-expressing *ycf 24* in *E.coli* was examined using the expresson vector pMAL-c2.

Gene amplification was carried out with primers desgned to give a 5' Eco RI site and a 3' Pst I site, and the product was cloned into the pMAL-c2 expression vector (New England Biolabs). Cultures of transfectants growing exponentially in Terrific broth containing ampicillin (50 mg ml⁻¹) were induced with 0.3-0.5 mM IPTG. Transformants expressing fusion proteins after induction at 37°C were detected by Western blotting and by immunofluorescence using antibody to the maltose binding protein tag of the fusion protein.

Filamentation was marked in such transformants.

Immunofluorescence showed a striking "zebra" pattern of the fusion protein which was mainly sequestered on either side of the nucleoids of the bacteria.

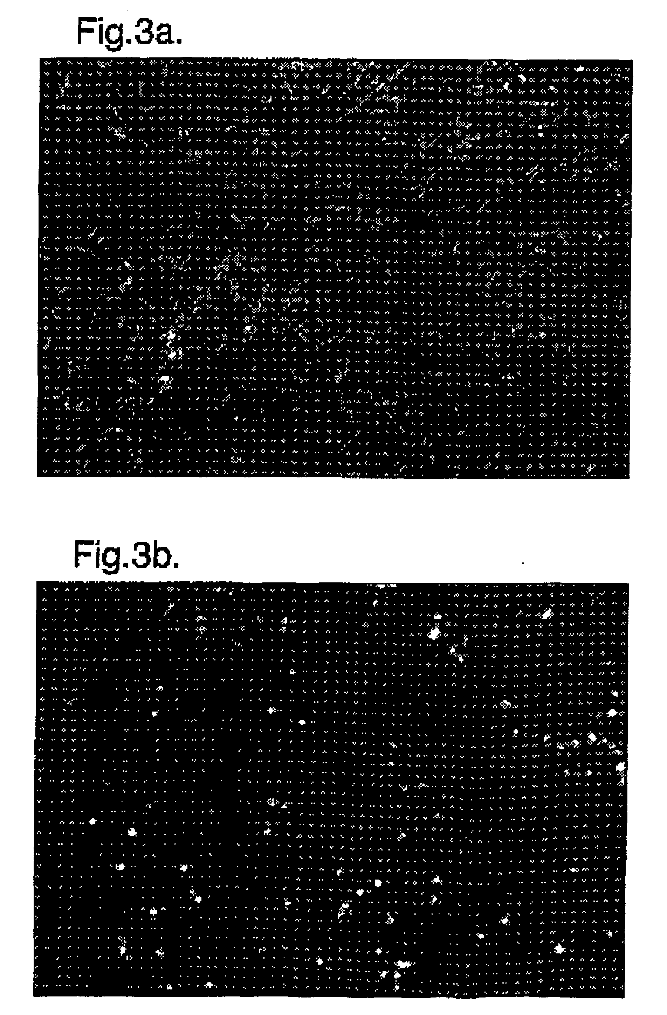

After prolonged over-expression, the bands of fusion protein appeared at other sites along the length of the extended cells where there were no nucleoids. This correlated with aberrant partition of the nucleoids. We found the nucleoids divided but did not separate, forming a clump that distorted the cell (see Fig. 3). We also noted that the first division of the nucleoids was parallel and not transverse to the cell length so that nucleoids lay side by side rather than being displaced longitudinally as is usual. Deletion analysis of the *ycf 24* gene showed that the "zebra" pattern due to deposition of the fusion protein depends on a sequence lying between amino acids 81 and 161 at the N-terminus of the protein. Secondary structure analysis of *ycf 24* indicates it probably has several domains, two of which lie in the highly conserved C-terminal half. One domain resembles a serine protease but lacks the catalytic site. Another, at the C-terminus itself, is folded like complement factor 5a.

Examination of the cells over-expressing *ycf 24* showed they contained only slightly depressed levels of the essential cell division protein FtsZ, yet no septal rings were formed (unlike in some other nucleoid partition mutants of *E.coli*). Thus, over-expression of *ycf 24* seems to disturb formation of the septal ring complex required for cell division.

Our studies with *E.coli* and *Synechocystis* concur in showing that modulating the amount of *ycf 24* affects cell division. Work is in progress to ascertain the function of the protein by determining its interactions with other cellular constituents. This includes a detailed analysis of the sequence required for cellular localization which might be susceptible to disruption by an antisense approach.

### References

1. Williamson, D.H., *et al. Mol. Gen. Genet.* **243,** 249-252(1994).
2. Denny, P., Preiser, P., Williamson, D. & Wilson, I. *Protist* **1**, 51-59 (1998).
3. Kowallik, K.V., Stoebe, B., Schaffran, I., Kroth-Pancic, P. & Freier, U. *Plant Mol. Report.* **13**, 336-342 (1995).
4. Wilson, R.J.M., *et al. J. Mol. Biol.* **261,** 155-172 (1996).
5. Palmer, J.D. *Nature* **364,** 762-763 (1993).
6. Wolfe, K.H., Morden, C.W. & Palmer, J.D. *Proc. Natl. Acad. Sci.* **89,** 10648-10652 (1992).
7. Labarre, J., Chauvat, F. & Thuriaux, P. *J. Bacteriol.* **171,** 3449-3457 (1989).
8. Paulton, R.J.L. *J. Bacteriol.* **104,** 762-767 (1970).
9. Walker, G.C. *The SOS response of Escherichia coli.* In scherichia coli and Salmonella Cellular and Molecular Biology. Vol.1 pp.1400-1416. (ASM Press, Washington D.C.) (1997).
10. Pietrokovski, S. *Prot. Sci.* **3,** 2340-2350 (1994).
11. Colston, M.J. & Davies, E.O. *Mol. Microbiol.* **12,** 359-363 (1994).
12. Jones, D.T., Taylor, W.R. & Thornton, J.M. *Nature* **358**, 86-89 (1992).
13. Fichera, M.E. & Roos, D.S. *Nature* **390,** 407-409 (1997).
14. McFadden, G.I. & Waller, R.F. *Bioessays* **19,** 1033-1040 (1997).
15. Hiraga, S., *et al. J. Bacteriol*. **171,** 1496-1505 (1989).
16. Blattner, F.R. *et al Science.* **277,**1453-1462 (1997)
17. Kaneko, T. *et al DNA Research.* **2,** 153-166 (1995)
18. Fichera, E.F. and Roos, D.S. *Nature* **390,** 407-409 (1997)
19. McConkey, G.A. *et al J. Biol. Chem.* **272,** 2046-2049 (1997)

## Claims

1. An antibody to the *ycf 24* gene product or an antisense inhibitor capable of hybridising with *ycf 24* mRNA for use in the treatment of infection of the human or animal body by an organism comprising said gene.

2. An antibody or antisense inhibitor according to claim 1 for use in the treatment of malaria.

3. A method of inhibiting the growth of an organism comprising the *ycf 24* gene, which method comprises contacting the organism *e*x *vivo* with an antibody to the *ycf 24* gene product or an antisense inhibitor capable of hybridising with *ycf 24* mRNA.

4. A method of identifying a compound that inhibits the growth of an organism comprising the *ycf 24* gene, the method comprising
(i) contacting a test compound with the *ycf 24* gene product, and
(ii) determining whether the test compound inhibits the activity of or binds to the product, any such binding or inhibition being indicative that the compound inhibits the growth of the organism.

5. A method of identifying a compound that inhibits the growth of an organism comprising the *ycf 24* gene, the method comprising
(i) contacting a test compound with a test construct comprising a *ycf 24* promoter operably linked to a coding sequence, and
(ii) determining whether the test compound inhibits expression driven by the promoter, any such inhibition being indicative that the compound inhibits the growth of the organism.

6. A method according to claim 4 or 5 in which the organism is a malaria parasite.

7. A pharmaceutical composition comprising an antibody to the *ycf 24* gene product or an antisense inhibitor capable of hybridising with *ycf 24* mRNA and a pharmaceutically acceptable carrier or diluent.

8. Use of an antibody to the *ycf 24* gene product or an antisense inhibitor capable of hybridising with *ycf 24* mRNA for the manufacture of a medicament for preventing or treating infection by a unicellular organism comprising the *ycf 24* gene in a patient.

9. A use according to claim 8 in which the organism is a malaria parasite.

## Patentansprüche

1. Antikörper gegen das *ycf24*-Genprodukt bzw. ein Antisense-Inhibitor, der in der Lage ist, mit der *ycf24*-RNA zu hybridisieren, zur Verwendung bei der Behandlung einer Infektion des Körpers von Menschen oder Tieren durch einen Organismus, der das Gen umfasst.

2. Antikörper bzw. ein Antisense-Inhibitor nach Anspruch 1 zur Verwendung in der Behandlung von Malaria.

3. Verfahren zum Hemmen des Wachstums eines Organismus umfassend das *ycf24*-Gen, wobei das Verfahren das In-Berührung-Bringen des Organismus ex *vivo* mit einem Antikörper gegen das *ycf24*-Genprodukt bzw. einem Antisense-Inhibitor umfasst, der in der Lage ist, mit der *ycf24*-RNA zu hybridisieren.

4. Verfahren zum Identifizieren einer Verbindung, die das Wachstum eines Organismus umfassend das *ycf24*-Gen hemmt, wobei das Verfahren umfasst
(i) In-Berührung-Bringen einer Testverbindung mit dem *ycf24*-Genprodukt, und
(ii) Bestimmen, ob die Testverbindung die Aktivität des Produktes hemmt oder an das Produkt bindet, wobei jede Bindung oder Hemmung anzeigt, dass die Verbindung das Wachstum des Organismus hemmt.

5. Verfahren zum Identifizieren einer Verbindung, die das Wachstum eines Organismus umfassend das *ycf24*-Gen hemmt, wobei das Verfahren umfasst
(i) In-Berührung-Bringen einer Testverbindung mit einem Testkonstrukt umfassend einen *ycf24*-Promotor, der funktional mit einer kodierenden Sequenz verbunden ist, und
(ii) Bestimmen, ob die Testverbindung die von dem Promotor angetriebene Expression hemmt, wobei jede Hemmung anzeigt, dass die Verbindung das Wachstum des Organismus hemmt.

6. Verfahren nach Anspruch 4 oder 5, in dem der Organismus ein Malariaparasit ist.

7. Pharmazeutische Zusammensetzung, umfassend einen Antikörper gegen das *ycf24*-Genprodukt bzw. einen Antisense-Inhibitor, der in der Lage ist, mit der *ycf24*-RNA zu hybridisieren, und einen pharmazeutisch akzeptablen Träger bzw. ein pharmazeutisch akzeptables Verdünnungsmittel.

8. Verwendung eines Antikörpers gegen das *ycf24*-Genprodukt bzw. einen Antisense-Inhibitor, der in der Lage ist, mit der *ycf24*-RNA zu hybridisieren, zur Herstellung eines Medikaments zum Verhindern oder Behandlung einer Infektion durch einen einzelligen Organismus umfassend das *ycf24*-Gen bei einem Patienten.

9. Verwendung nach Anspruch 8, in dem der Organismus ein Malariaparasit ist.

## Revendications

1. Anticorps contre le produit du gène *ycf 24* ou inhibiteur antisens capable de s'hybrider avec l'ARNm de *ycf 24,* destiné à être utilisé dans le traitement d'une infection de l'organisme humain ou animal par un organisme comprenant ledit gène.

2. Anticorps ou inhibiteur antisens selon la revendication 1, destiné à être utilisé dans le traitement de la malaria.

3. Méthode d'inhibition de la croissance d'un organisme comprenant le gène *ycf 24,* cette méthode comprenant le fait de mettre en contact l'organisme *ex vivo* avec un anticorps contre le produit du gène *ycf 24* ou un inhibiteur antisens capable de s'hybrider avec l'ARNm de *ycf 24*.

4. Méthode d'identification d'un composé qui inhibe la croissance d'un organisme comprenant le gène *ycf 24,* la méthode comprenant le fait de
(i) mettre en contact un composé à tester avec le produit du gène *ycf 24,* et
(ii) déterminer si le composé à tester inhibe l'activité ou se lie au produit, une telle liaison ou inhibition quelle qu'elle soit indiquant que le composé inhibe la croissance de l'organisme.

5. Méthode d'identification d'un composé qui inhibe la croissance d'un organisme comprenant le gène *ycf 24,* la méthode comprenant le fait de
(i) mettre en contact un composé à tester avec une construction de test comprenant un promoteur de *ycf 24* lié de façon opérationnel à une séquence codante, et
(ii) déterminer si le composé à tester inhibe l'expression dirigée par le promoteur, une telle inhibition quelle qu'elle soit indiquant que le composé inhibe la croissance de l'organisme.

6. Méthode selon la revendication 4 ou 5, dans laquelle l'organisme est un parasite malarique.

7. Composition pharmaceutique comprenant un anticorps contre le produit du gène *ycf 24* ou un inhibiteur antisens capable de s'hybrider avec l'ARNm de *ycf 24* et un véhicule ou un diluant pharmaceutiquement acceptable.

8. Utilisation d'un anticorps contre le produit du gène *ycf 24* ou d'un inhibiteur antisens capable de s'hybrider avec l'ARNm de *ycf 24* pour la fabrication d'un médicament pour prévenir ou traiter une infection par un organisme unicellulaire comprenant le gène *ycf 24* chez un patient.

9. Utilisation selon la revendication 8, dans laquelle l'organisme est un parasite malarique.


### SEQUENCE LISTING

<210> 1
   <211> 1444
   <212> DNA
   <213> Plasmodium falciparum
   <220>
   <221> CDS
   <222> (26)..(1435)
   <220>
   <221> misc_feature
   <222> (1276)
   <223> h represents A, T or C
<400> 1
   tttattattt ttatatataa ttatt atg ata aaa tta aaa aat ttt tta aat    52
                               Met Ile Lys Leu Lys Asn Phe Leu Asn
                                1              5
   att tat aat tta aat tat aaa tat caa tat aaa aat aaa ata aat tta   100
   Ile Tyr Asn Leu Asn Tyr Lys Tyr Gln Tyr Lys Asn Lys Ile Asn Leu
    10                  15                  20                  25
   tat tta ata aga caa gga tta aat ata aat tta ata aaa aat tta tct   148
   Tyr Leu Ile Arg Gln Gly Leu Asn Ile Asn Leu Ile Lys Asn Leu Ser
                    30                  35                  40
   agt aat att ttt tta tat atg ttt att tat aat ttt aaa aaa tat tct   196
   Ser Asn Ile Phe Leu Tyr Met Phe Ile Tyr Asn Phe Lys Lys Tyr Ser
                45                  50                  55
   tta aaa tta tta aat ata ttt aaa tta cct gat tgg aat ttt ttt gat   244
   Leu Lys Leu Leu Asn Ile Phe Lys Leu Pro Asp Trp Asn Phe Phe Asp
            60                  65                  70
   tgt cca aat ata aat tat gat aat att att tat tat tct tct att tta   292
   Cys Pro Asn Ile Asn Tyr Asp Asn Ile Ile Tyr Tyr Ser Ser Ile Leu
        75                  80                  85
   aaa gat aat aat tta ata tat tat tta aaa aat aat tta aat att gaa   340
   Lys Asp Asn Asn Leu Ile Tyr Tyr Leu Lys Asn Asn Leu Asn Ile Glu
    90                  95                 100                 105
   ttt tta gat agt ata tta ata aaa aat aat tct ata gat att ata ttt   388
   Phe Leu Asp Ser Ile Leu Ile Lys Asn Asn Ser Ile Asp Ile Ile Phe
                   110                 115                 120
   gat agt atg tct att tta cat act aca caa tat ttt tta aaa aaa tth   436
   Asp Ser Met Ser Ile Leu His Thr Thr Gln Tyr Phe Leu Lys Lys Xaa
               125                 130                 135
   gga ata att ttt tta cct tta ttt gat att ata ttt aaa tat cct tta    484
   Gly Ile Ile Phe Leu Pro Leu Phe Asp Ile Ile Phe Lys Tyr Pro Leu
           140                 145                 150
   tta ata aaa aaa tat tta ggt act att att tct tat aaa gat aat ttt    532
   Leu Ile Lys Lys Tyr Leu Gly Thr Ile Ile Ser Tyr Lys Asp Asn Phe
       155                 160                 165
   ttt gct aat att aat tca ata ata ttt agt gaa gga tct ttt tgt tat    580
   Phe Ala Asn Ile Asn Ser Ile Ile Phe Ser Glu Gly Ser Phe Cys Tyr
   170                 175                 180                 185
   ata cct aaa tat gta aag tgt aat ttt aat tta tca aca tat ttt aaa    628
   Ile Pro Lys Tyr Val Lys Cys Asn Phe Asn Leu Ser Thr Tyr Phe Lys
                   190                 195                 200
   act aat tct tct gat ttt gca caa ttt gaa cgt act tta ata ata gtt    676
   Thr Asn Ser Ser Asp Phe Ala Gln Phe Glu Arg Thr Leu Ile Ile Val
               205                 210                 215
   ggt aaa tat tct tat gta tca tat tta gaa gga tgt aca gct tca tta    724
   Gly Lys Tyr Ser Tyr Val Ser Tyr Leu Glu Gly Cys Thr Ala Ser Leu
           220                 225                 230
   tat aaa gaa tca caa tta cat gta gct ata gta gaa ata ata gta aaa    772
   Tyr Lys Glu Ser Gln Leu His Val Ala Ile Val Glu Ile Ile Val Lys
       235                 240                 245
   gat tat ggt tat ata aaa tat tat aca tta caa aat tgg tat aga gga    820
   Asp Tyr Gly Tyr Ile Lys Tyr Tyr Thr Leu Gln Asn Trp Tyr Arg Gly
   250                 255                 260                 265
   gat tat tta ggt aat ggt ggt tta tat aat ttt aca act aaa cgt ggt    868
   Asp Tyr Leu Gly Asn Gly Gly Leu Tyr Asn Phe Thr Thr Lys Arg Gly
                   270                 275                 280
   ata tgt tta aat tat tca aaa tta gat tgg ata caa gtt gaa gta ggt    916
   Ile Cys Leu Asn Tyr Ser Lys Leu Asp Trp Ile Gln Val Glu Val Gly
               285                 290                 295
   tcg att ata aca tgg aaa tac cct tct act att tta aaa ggt aaa ttt    964
   Ser Ile Ile Thr Trp Lys Tyr Pro Ser Thr Ile Leu Lys Gly Lys Phe
           300                 305                 310
   tct att agt aat ttt tat tca ata tct ttt ata tca aat atg caa ata    1012
   Ser Ile Ser Asn Phe Tyr Ser Ile Ser Phe Ile Ser Asn Met Gln Ile
       315                 320                 325
   gct gat act ggt agt aaa atg tat cat ata gga tct tat act aaa agt    1060
   Ala Asp Thr Gly Ser Lys Met Tyr His Ile Gly Ser Tyr Thr Lys Ser
   330                 335                 340                 345
   tat ata att tct aaa agt ata tct tta aat aac tca tta aat ata ttt    1108
   Tyr Ile Ile Ser Lys Ser Ile Ser Leu Asn Asn Ser Leu Asn Ile Phe
                   350                 355                 360
   aga ggt tta gta tat att aaa cct ttt tca tat aaa tct tat aat tat    1156
   Arg Gly Leu Val Tyr Ile Lys Pro Phe Ser Tyr Lys Ser Tyr Asn Tyr
                   365                 370                 375
   act gaa tgt agt tct tta ata ttt ggt aat aat tct tta aca gta act    1204
   Thr Glu Cys Ser Ser Leu Ile Phe Gly Asn Asn Ser Leu Thr Val Thr
               380                 385                 390
   att cct tat ata aaa aat tat aat aat act agt tat gta aaa caa gaa    1252
   Ile Pro Tyr Ile Lys Asn Tyr Asn Asn Thr Ser Tyr Val Lys Gln Glu
           395                 400                 405
   gct ttt gtt tct aaa att gaa ath ata tat tta ttt tta tta atg caa    1300
   Ala Phe Val Ser Lys Ile Glu Ile Ile Tyr Leu Phe Leu Leu Met Gln
   410                 415                 420                 425
   cgt ggt tta agt att tca gag tct att tca tta tta att ata ggt ttt    1348
   Arg Gly Leu Ser Ile Ser Glu Ser Ile Ser Leu Leu Ile Ile Gly Phe
                   430                 435                 440
   tgt tct gat att tat aat aaa tta ccg ttt gaa ttt aat tta gag ata    1396
   Cys Ser Asp Ile Tyr Asn Lys Leu Pro Phe Glu Phe Asn Leu Glu Ile
               445                 450                 455
   cct ata tta ttt tca tta aaa att aaa gat ata ttt aat taattaaat      1444
   Pro Ile Leu Phe Ser Leu Lys Ile Lys Asp Ile Phe Asn
           460                 465                 470

<210> 2
   <211> 1443
   <212> DNA
   <213> Synechocystis PCC6803
   <220>
   <221> CDS
   <222> (1)..(1443)
<400> 2
   atg agt tcc acc act gtt aaa aac ctg gtc aac caa ccc tac aaa tat    48
   Met Ser Ser Thr Thr Val Lys Asn Leu Val Asn Gln Pro Tyr Lys Tyr
     1               5                  10                  15
   ggc ttt gtc acc aac att gaa gcg gat gct atc ccc cgt ggt ctg agt    96
   Gly Phe Val Thr Asn Ile Glu Ala Asp Ala Ile Pro Arg Gly Leu Ser
                    20                  25                  30
   gaa gac gtg gtg cga ctc att tct gct aag aaa aat gaa ccc gaa ttc    144
   Glu Asp Val Val Arg Leu Ile Ser Ala Lys Lys Asn Glu Pro Glu Phe
                35                  40                  45
   atg ttg gat ttt cgc ctc cgg gcc tac cgg cat tgg ctg acc atg gcg    192
   Met Leu Asp Phe Arg Leu Arg Ala Tyr Arg His Trp Leu Thr Met Ala
            50                  55                  60
   gaa ccc act tgg ccg gcg gtg cat tat ccc ccc att gat tac caa gat    240
   Glu Pro Thr Trp Pro Ala Val His Tyr Pro Pro Ile Asp Tyr Gln Asp
   65                  70                  75                  80
   att att tac tac tcc gcc cct aag caa agt aag aaa aaa cta gaa agc    288
   Ile Ile Tyr Tyr Ser Ala Pro Lys Gln Ser Lys Lys Lys Leu Glu Ser
                   85                  90                  95
   tta gat gaa gtg gac cca gct ttg ttg gaa acc ttt gaa aaa tta ggg    336
   Leu Asp Glu Val Asp Pro Ala Leu Leu Glu Thr Phe Glu Lys Leu Gly
               100                 105                 110
   att ccc cta tcg gag caa aaa cgt tta agt aat gtg gcg gta gat gcc    384
   Ile Pro Leu Ser Glu Gln Lys Arg Leu Ser Asn Val Ala Val Asp Ala
           115                 120                 125
   att ttt gac agt gtt tcc att ggc aca act ttt aag gaa aag cta gcg    432
   Ile Phe Asp Ser Val Ser Ile Gly Thr Thr Phe Lys Glu Lys Leu Ala
       130                 135                 140
   gaa gac ggg gta att ttc tgt tct att tct gaa gca ttg cag gaa cat    480
   Glu Asp Gly Val Ile Phe Cys Ser Ile Ser Glu Ala Leu Gln Glu His
   145                 150                 155                 160
   ccc gac ctg gtg caa aaa tat ttg ggc agt gtg gtg ccc acc gcc gac    528
   Pro Asp Leu Val Gln Lys Tyr Leu Gly Ser Val Val Pro Thr Ala Asp
                   165                 170                 175
   aac ttc ttt gcc gcc tta aac tct gct gta ttt agt gac ggt tcc ttt    576
   Asn Phe Phe Ala Ala Leu Asn Ser Ala Val Phe Ser Asp Gly Ser Phe
               180                 185                 190
   gtt ttt att ccc aaa ggg gtg aag tgt ccc atg gaa ttg tcc acc tat    624
   Val Phe Ile Pro Lys Gly Val Lys Cys Pro Met Glu Leu Ser Thr Tyr
           195                 200                 205
   ttc cgc att aat aat ggg gat acg ggg cag ttt gag cgg aca tta att    672
   Phe Arg Ile Asn Asn Gly Asp Thr Gly Gln Phe Glu Arg Thr Leu Ile
       210                 215                 220
   att gcc gaa gaa ggg gct tcc gtt agc tat ttg gaa ggt tgt act gcg    720
   Ile Ala Glu Glu Gly Ala Ser Val Ser Tyr Leu Glu Gly Cys Thr Ala
   225                 230                 235                 240
   ccc atg tat gac acc aat caa ctt cat gcg gcg gtg gtg gaa ttg gta    768
   Pro Met Tyr Asp Thr Asn Gln Leu His Ala Ala Val Val Glu Leu Val
                   245                 250                 255
   gct cta gat aat gct gac att aaa tat tcc acc gta caa aac tgg tac    816
   Ala Leu Asp Asn Ala Asp Ile Lys Tyr Ser Thr Val Gln Asn Trp Tyr
               260                 265                 270
   gct ggg gac gaa aat ggc aag ggc gga att tac aac ttt gtg act aaa    864
   Ala Gly Asp Glu Asn Gly Lys Gly Gly Ile Tyr Asn Phe Val Thr Lys
           275                 280                 285
   cgg ggt cta tgt aaa gga gtt aat tcc aaa att tcc tgg acc caa gta   912
   Arg Gly Leu Cys Lys Gly Val Asn Ser Lys Ile Ser Trp Thr Gln Val
       290                 295                 300
   gaa acc ggt tcc gcc att acc tgg aaa tac ccc agt tgt gtg cta gtt   960
   Glu Thr Gly Ser Ala Ile Thr Trp Lys Tyr Pro Ser Cys Val Leu Val
   305                 310                 315                 320
   ggg gat aat tcc gtc ggg gaa ttc tac tct att gct tta act aac aac   1008
   Gly Asp Asn Ser Val Gly Glu Phe Tyr Ser Ile Ala Leu Thr Asn Asn
                   325                 330                 335
   aaa cag caa gct gat acg gga act aaa atg att cac atc ggt aaa aat   1056
   Lys Gln Gln Ala Asp Thr Gly Thr Lys Met Ile His Ile Gly Lys Asn
               340                 345                 350
   acc cgt agt atc att att tcc aaa ggc att tcc gct ggt aat tcc gcc   1104
   Thr Arg Ser Ile Ile Ile Ser Lys Gly Ile Ser Ala Gly Asn Ser Ala
           355                 360                 365
   aac agt tac cgg ggt ttg gtg aaa atg gga cct aaa gcc cag ggc gct   1152
   Asn Ser Tyr Arg Gly Leu Val Lys Met Gly Pro Lys Ala Gln Gly Ala
       370                 375                 380
   cgc aat tat tcc cag tgt gat tcc atg ctc att ggc gat cgg gca gcg   1200
   Arg Asn Tyr Ser Gln Cys Asp Ser Met Leu Ile Gly Asp Arg Ala Ala
   385                 390                 395                 400
   gct aat act ttt ccc tat att caa gtg gac aat aat acc gcc aaa gta   1248
   Ala Asn Thr Phe Pro Tyr Ile Gln Val Asp Asn Asn Thr Ala Lys Val
                   405                 410                 415
   gaa cat gaa gct tcc act tcc aaa att ggc gag gat caa ctc ttt tac   1296
   Glu His Glu Ala Ser Thr Ser Lys Ile Gly Glu Asp Gln Leu Phe Tyr
               420                 425                 430
   ttt gcc caa cgg gga att tct gag gaa gat gcg gtg tcc atg cta gtc   1344
   Phe Ala Gln Arg Gly Ile Ser Glu Glu Asp Ala Val Ser Met Leu Val
           435                 440                 445
   agc ggt ttc tgt aag gat gtg cta aac gaa tta ccc atg gaa ttt gcg   1392
   Ser Gly Phe Cys Lys Asp Val Leu Asn Glu Leu Pro Met Glu Phe Ala
       450                 455                 460
   gcg gag gct gat aaa tta ctg agt ctc aaa cta gaa ggt act gtg ggt   1440
   Ala Glu Ala Asp Lys Leu Leu Ser Leu Lys Leu Glu Gly Thr Val Gly
   465                 470                 475                 480
   taa                                                               1443

<210> 3
   <211> 1527
   <212> DNA
   <213> Escherichia coli
   <220>
   <221> CDS
   <222> (1)..(1527)
<400> 3
   atg tgg ctg tgg cga aag ctt tgg ggt ata ggc ggt act atg tct cgt 48
   Met Trp Leu Trp Arg Lys Leu Trp Gly Ile Gly Gly Thr Met Ser Arg
   1 5 10 15
   aat act gaa gca act gac gat gtc aaa acc tgg acc ggc ggc ccg ctg 96
   Asn Thr Glu Ala Thr Asp Asp Val Lys Thr Trp Thr Gly Gly Pro Leu
   20 25 30
   aat tat aaa gaa gga ttc ttc acc cag tta gcc acc gat gag ctg gca 144
   Asn Tyr Lys Glu Gly Phe Phe Thr Gln Leu Ala Thr Asp Glu Leu Ala
   35 40 45
   aag ggg ata aac gaa gag gtg gtg cgc gca att tcg gcg aag cgt aat 192
   Lys Gly Ile Asn Glu Glu Val Val Arg Ala Ile Ser Ala Lys Arg Asn
   50 55 60
   gag ccg gag tgg atg ctg gag ttt cgt cta aac gcc tat cgc gca tgg 240
   Glu Pro Glu Trp Met Leu Glu Phe Arg Leu Asn Ala Tyr Arg Ala Trp
   65 70 75 80
   ctg gag atg gaa gaa ccg cac tgg ttg aaa gcg cac tac gac aag ctg 288
   Leu Glu Met Glu Glu Pro His Trp Leu Lys Ala His Tyr Asp Lys Leu
   85 90 95
   aat tat cag gat tac agc tac tac tca gca cca tcg tgc ggt aat tgt 336
   Asn Tyr Gln Asp Tyr Ser Tyr Tyr Ser Ala Pro Ser Cys Gly Asn Cys
   100 105 110
   gac gac act tgc gcg tct gaa cct ggc gcg gtg cag caa act ggc gcg 384
   Asp Asp Thr Cys Ala Ser Glu Pro Gly Ala Val Gln Gln Thr Gly Ala
   115 120 125
   aac gcc ttt tta agt aaa gag gtg gag gcg gcg ttt gag cag ttg ggc 432
   Asn Ala Phe Leu Ser Lys Glu Val Glu Ala Ala Phe Glu Gln Leu Gly
   130 135 140
   gtt ccc gtg cgg gaa ggc aaa gag gtg gcg gtg gat gcc att ttc gac 480
   Val Pro Val Arg Glu Gly Lys Glu Val Ala Val Asp Ala Ile Phe Asp
   145 150 155 160
   tca gtt tcg gtt gcc act act tat cgc gaa aaa ctg gcg gag cag gga 528
   Ser Val Ser Val Ala Thr Thr Tyr Arg Glu Lys Leu Ala Glu Gln Gly
   165 170 175
   att att ttc tgt tcc ttt ggt gag gcg atc cac gat cac ccg gaa ctg 576
   Ile Ile Phe Cys Ser Phe Gly Glu Ala Ile His Asp His Pro Glu Leu
   180 185 190
   gtg cgt aaa tat ctc ggc acc gtg gtg ccg ggg aat gac aac ttc ttt 624
   Val Arg Lys Tyr Leu Gly Thr Val Val Pro Gly Asn Asp Asn Phe Phe
   195 200 205
   gcc gcg ctt aat gcg gcg gta gcc tct gat ggt acg ttt att tat gtg    672
   Ala Ala Leu Asn Ala Ala Val Ala Ser Asp Gly Thr Phe Ile Tyr Val
       210                 215                 220
   cct aaa ggc gtg cgc tgc ccg atg gaa ctt tcc acc tat ttt cgc att    720
   Pro Lys Gly Val Arg Cys Pro Met Glu Leu Ser Thr Tyr Phe Arg Ile
   225                 230                 235                 240
   aac gca gaa aaa acc ggg cag ttt gag cgc acc att ctg gtg gcc gac    768
   Asn Ala Glu Lys Thr Gly Gln Phe Glu Arg Thr Ile Leu Val Ala Asp
                   245                 250                 255
   gaa gac agc tac gtc agc tac att gaa ggc tgt tcc gct ccg gtg cgt    816
   Glu Asp Ser Tyr Val Ser Tyr Ile Glu Gly Cys Ser Ala Pro Val Arg
               260                 265                 270
   gac agc tat cag tta cac gcg gca gtg gtg gaa gtc atc atc cat aaa    864
   Asp Ser Tyr Gln Leu His Ala Ala Val Val Glu Val Ile Ile His Lys
           275                 280                 285
   aac gcc gag gtg aaa tat tcc acg gta caa aac tgg ttt cct ggc gat    912
   Asn Ala Glu Val Lys Tyr Ser Thr Val Gln Asn Trp Phe Pro Gly Asp
       290                 295                 300
   aac aac acc ggc ggt att ctc aac ttc gtc acc aag cgt gct ttg tgc    960
   Asn Asn Thr Gly Gly Ile Leu Asn Phe Val Thr Lys Arg Ala Leu Cys
   305                 310                 315                 320
   gaa ggc gaa aac agc aaa atg tca tgg acg caa tca gaa acc ggg tca    1008
   Glu Gly Glu Asn Ser Lys Met Ser Trp Thr Gln Ser Glu Thr Gly Ser
                   325                 330                 335
   gcg att acg tgg aaa tat ccc agc tgc att ttg cgc ggc gat aac tcc    1056
   Ala Ile Thr Trp Lys Tyr Pro Ser Cys Ile Leu Arg Gly Asp Asn Ser
               340                 345                 350
   att ggt gag ttt tac tca gtg gcg ctg acc agc ggt cat cag caa gcg    1104
   Ile Gly Glu Phe Tyr Ser Val Ala Leu Thr Ser Gly His Gln Gln Ala
           355                 360                 365
   gat acc ggc acc aag atg atc cac atc ggt aaa aac acc aaa tcg acc    1152
   Asp Thr Gly Thr Lys Met Ile His Ile Gly Lys Asn Thr Lys Ser Thr
       370                 375                 380
   att atc tcg aaa ggg atc tct gcc gga cat agt cag aac agt tat cgc    1200
   Ile Ile Ser Lys Gly Ile Ser Ala Gly His Ser Gln Asn Ser Tyr Arg
   385                 390                 395                 400
   ggc tta gtg aaa atc atg ccg acg gca acc aat gcg cgc aat ttc act    1248
   Gly Leu Val Lys Ile Met Pro Thr Ala Thr Asn Ala Arg Asn Phe Thr
                   405                 410                 415
   cag tgc gac tca atg ctg att ggc gct aat tgt ggg gcg cat acc ttc    1296
   Gln Cys Asp Ser Met Leu Ile Gly Ala Asn Cys Gly Ala His Thr Phe
               420                 425                 430
   ccg tat gtt gag tgt cgt aac aat agt gcg caa ctg gaa cac gag gca    1344
   Pro Tyr Val Glu Cys Arg Asn Asn Ser Ala Gln Leu Glu His Glu Ala
           435                 440                 445
   acg aca tca cgt att ggt gaa gat caa ctg ttt tac tgc ctg caa cgc    1392
   Thr Thr Ser Arg Ile Gly Glu Asp Gln Leu Phe Tyr Cys Leu Gln Arg
       450                 455                 460
   ggg atc agc gaa gaa gac gcc atc tcg atg att gtt aac ggt ttc tgc    1440
   Gly Ile Ser Glu Glu Asp Ala Ile Ser Met Ile Val Asn Gly Phe Cys
   465                 470                 475                 480
   aaa gac gtg ttc tcg gag ctg ccg ttg gaa ttt gcc gtt gaa gca caa    1488
   Lys Asp Val Phe Ser Glu Leu Pro Leu Glu Phe Ala Val Glu Ala Gln
                   485                 490                 495
   aaa ctc ctc gcc atc agt ctt gaa cac agc gtc gga taa                1527
   Lys Leu Leu Ala Ile Ser Leu Glu His Ser Val Gly
               500                 505